## Europäisches Patentamt
## European Patent Office
## Office européen des brevets

(19)

(11) Veröffentlichungsnummer: **0 027 979**
**B1**

(12) # EUROPÄISCHE PATENTSCHRIFT

(45) Veröffentlichungstag der Patentschrift:
**23.06.82**

(21) Anmeldenummer: **80106358.7**

(22) Anmeldetag: **18.10.80**

(51) Int. Cl.³: **C 07 C 67/055**, C 07 C 69/16, C 07 C 69/18, B 01 J 23/89

(54) **Verfahren zur Herstellung von Acyloxybutenen.**

(30) Priorität: **26.10.79 DE 2943407**

(43) Veröffentlichungstag der Anmeldung:
**06.05.81 Patentblatt 81/18**

(45) Bekanntmachung des Hinweises auf die Patenterteilung:
**23.06.82 Patentblatt 82/25**

(84) Benannte Vertragsstaaten:
**BE CH DE FR GB IT LI NL**

(56) Entgegenhaltungen:
**DE-A-2 444 004**
**DE-A-2 747 634**
**DE-A-2 820 519**
**DE-A-2 842 238**

(73) Patentinhaber: **BASF Aktiengesellschaft, Carl-Bosch-Strasse 38, D-6700 Ludwigshafen (DE)**

(72) Erfinder: **Weitz, Hans-Martin, Dr., Auf dem Koeppel 40, D-6702 Bad Duerkeheim 1 (DE)**
Erfinder: **Fischer, Rolf, Dr., Bergstrasse 98, D-6900 Heidelberg (DE)**
Erfinder: **Schnabel, Rolf, Dr., Tilsiter Strasse 9, D-6707 Schifferstadt (DE)**

**0 027 979**

### Verfahren zur Herstellung von Acyloxybutenen

Die Erfindung betrifft ein Verfahren zur Herstellung von Acyloxybutenen, wie 1.2- und 1.4-Acyloxybutenen durch Umsetzung von ggf. substituiertem 1.3-Butadien mit Sauerstoff und niedermolekularen Carbonsäuren in Gegenwart eines Palladium oder Platin und Tellur enthaltenden Trägerkatalysators, der eine intermetallische Palladium-Kupfer- oder Platin-Kupfer-Verbindung enthält.

Bekanntlich lassen sich 1.3-Butadiene mit Sauerstoff und Carbonsäuren in Gegenwart von gelösten Palladium- und Kupfersalzen zu 1.2- und 1.4-Butendiolestern umsetzen. Nach den Angaben der GB-PS 1 138 366 erhält man durch Umsetzung von Piperylen mit Luft in Gegenwart von in Eisessig gelöstem Palladiumacetat, Kupferacetat und Lithiumacetat Gemische aus 1.4-Diacetoxy-2-penten und 4.5-Diacetoxy-2-penten (Molverhältnis 4 : 1). In ähnlicher Weise wird Butadien nach der GB-PS 1 277 837 mit Sauerstoff in Gegenwart von in Eisessig gelöstem Palladiumacetat, Kupferacetat und Kaliumacetat zu 1.4-Diacetoxy-2-buten umgesetzt. Eine Variante dieser Katalysatorkombination wird in der japanischen Patentanmeldung 19 293 (1973) genannt, in der angegeben ist, daß die Ausbeute durch Zugabe von Acetanhydrid erhöht werden kann. In der japanischen Patentanmeldung 13 714 (1976) werden für die Umsetzung von Butadien mit Essigsäure und Sauerstoff Lösungen von Palladiumacetat, Kupferacetat und Lithiumchlorid vorgeschlagen.

Es ist weiterhin schon vorgeschlagen worden, die Umsetzung von Butadien mit Essigsäure und Sauerstoff (»Acetoxylierung«) in Gegenwart von metallisches Palladium enthaltenden festen Katalysatoren mit Aktivkohle als Träger durchzuführen, wobei diese Katalysatoren in Lösungen von Kupferacetat und Lithiumacetat in Eisessig und Acetanhydrid (japanische Patentanmeldung 126 607 [1975]) von Kupferchlorid, Lithiumacetat und Antimontrichlorid (japanische Patentanmeldung 39 004 [1972]) oder von Kupferchlorid in Acetanhydrid (japanische Patentanmeldung 39 003 [1972]) suspendiert wurden. Bei der in der DE-PS 2 012 903 beschriebenen Acetoxylierung von Butadien werden metallisches Palladium enthaltende Katalysatoren verwendet, bei deren Herstellung Kupfersalze, wie Kupferchlorid oder Kupferacetat zugegeben werden.

Einer technischen Nutzung dieser Verfahren steht entgegen, daß die Katalysatoren nur einen niedrigen Butadien-Umsatz bewirken und mit geringer Selektivität arbeiten. Weiterhin ist die Verwendung des teuren Acetanhydrids von Nachteil, auch ist es schwierig, gewisse homogen gelöste Katalysatorkomponenten abzutrennen, gegebenenfalls aufzuarbeiten und in den Prozeß zurückzuführen.

In der DE-PS 2 217 452 wird ein Verfahren zur Acetoxylierung von Butadien beschrieben, bei dem man Palladium-Katalysatoren verwendet, die zusätzlich Antimon, Wismut, Tellur oder Selen enthalten, und die in der DE-OS 2 728 574 beschriebene Acetoxylierung führt man in Gegenwart von schwefelhaltigen Palladium-Katalysatoren durch, die außerdem noch andere Elemente enthalten können. Bei Verwendung dieser Katalysatoren erhält man Ergebnisse, die z. B. hinsichtlich der Raum-Zeit-Ausbeute nicht befriedigen.

Es wurde nun gefunden, daß man bei der Herstellung von Acyloxybutenen durch Umsetzung von ggf. substituiertem 1.3-Butadien mit Sauerstoff und niedermolekularen Carbonsäuren in Gegenwart eines Palladium oder Platin und Tellur enthaltenden Trägerkatalysators besonders vorteilhafte Ergebnisse erzielt, wenn der Katalysator eine intermetallische Palladium-Kupfer- oder Platin-Kupfer-Verbindung enthält.

Nach dem neuen Verfahren erhält man hohe Raum-Zeit-Ausbeuten. Die nach dem erfindungsgemäßen Verfahren zu verwendenden Katalysatoren weisen eine hohe Standzeit auf, sie erweisen sich auch hochselektiv in bezug auf die erwünschte Bildung von Butendioldiacetaten und -monoacetaten.

Der nach der Erfindung zu verwendende Trägerkatalysator enthält als aktive Bestandteile, die auf dem Trägermaterial aufgebracht sind, Palladium oder Platin und Kupfer sowie Tellur. Der Katalysator ist ferner dadurch gekennzeichnet, daß er eine intermetallische Palladium-Kupfer- oder Platin-Kupfer-Verbindung enthält. Solche intermetallische Phasen, die die Zusammensetzung $PdCu_3$, $PtCu_3$ oder PdCu, PtCu haben, sind röntgengraphisch nachweisbar. Intermetallische Palladium-Tellur- oder Platin-Tellur-Phasen lassen sich durch Röntgenanalyse des erfindungsgemäß verwendeten Katalysators nicht feststellen.

Der Katalysator der genannten Art enthält beispielsweise, bezogen auf das Katalysatorgewicht, 1 bis 10% Palladium oder Platin, 0,1 bis 30% Kupfer und 0,01 bis 10% Tellur. Bevorzugt ist die Verwendung eines Trägerkatalysators, der je Grammatom Palladium oder Platin 0,01 bis 6, vorzugsweise 1 bis 3,5 Grammatome Kupfer und 0,01 bis 1, vorzugsweise 0,01 bis 0,4 Grammatome Tellur, enthält.

Die Gesamtmenge der auf den Träger aufgebrachten katalytisch wirksamen Metalle beträgt, bezogen auf den Trägerkatalysator, zweckmäßig z. B. 0,01 bis 30 Gew.-%. Größere und kleinere Konzentrationen sind allerdings möglich. Die Entscheidung, welche Menge die günstigste ist, kann auch von wirtschaftlichen Überlegungen bestimmt werden.

Als Trägermaterial kommen für den Katalysator z. B. Aktivkohle, Bauxit, Bimsstein, Kieselgel, Kieselgur oder andere Formen der Kieselsäure, Magnesia, Ton, Tonerde in Betracht. Die Träger

**0 027 979**

können durch eine übliche Vorbehandlung, z. B. mit einer Säure, für ihre Zweckbestimmung verbessert werden.

Für die Herstellung des Katalysators ist die zu wählende Palladium- oder Platin-Verbindung nicht entscheidend. So kann man z. B. halogenierte Palladium- oder Platin-Verbindungen, wie Palladium- oder Platinchlorid, ein Salz einer organischen Säure, wie Palladiumacetat oder Platinacetat, die Nitrate oder Oxide verwenden. Man kann aber auch von anderen Verbindungen, insbesondere komplexen Verbindungen, ausgehen. Auch Kupfer und Tellur können weitgehend in Form frei wählbarer Verbindungen in die herzustellenden Katalysatoren eingeführt werden. Aus Zweckmäßigkeitsgründen wird man zu löslichen Verbindungen greifen.

Der Katalysator wird z. B. hergestellt, indem man den Träger in einer Lösung dispergiert, die eine Palladium- oder Platinverbindung sowie eine Kupfer- und Tellurverbindung enthält, das Lösungsmittel verdampft und den Rückstand in einem Gasstrom aus z. B. Wasserstoff oder Stickstoff, der mit einer reduzierenden Verbindung, wie Hydrazin, Methanol oder Formaldehyd beladen ist, reduziert. Die Reduktion des getrockneten Katalysators kann auch mit flüssigen Reduktionsmitteln geschehen. Man kann den Katalysator auch herstellen, indem man Träger und Lösung gemeinsam mit einem z. B. alkalischen Fällungsmittel behandelt und die Ausfällung gewinnt und reduziert. Eine sehr brauchbare Methode zur Herstellung der Katalysatoren ist die Fällung der Metalle aus wäßrigen Salzlösungen, unmittelbar durch Reduktionsmittel, wie Formaldehyd, Hydrazin bei einem geeigneten pH-Wert (siehe z. B. JACS, 83 [1961], S. 4916).

Zweckmäßig werden die so erhaltenen rohen Katalysatoren noch in einem reduzierend wirkenden Gasstrom auf höhere Temperaturen erhitzt.

Palladium oder Platin, Kupfer und Tellur können gleichzeitig oder in beliebiger Reihenfolge nacheinander auf dem Träger abgeschieden werden; in manchen Fällen kann der Träger in Form einer löslichen Verbindung zugefügt und gemeinsam mit dem wirksamen Metall ausgefällt werden.

Man kann jedes Reduktionsverfahren anwenden, durch welches die verwendeten Elemente in den metallischen Zustand überführt werden.

Es ist erforderlich, daß der nach diesen an sich bekannten Verfahren erhältliche Katalysator, bei dem die Metalle in Form zufälliger physikalischer Mischungen vorliegen, in die beim Verfahren der Erfindung benötigte Form gebracht, d. h. in einen Katalysator übergeführt wird, der eine intermetallische Palladium-Kupfer- oder Platin-Kupfer-Verbindung enthält.

Intermetallische Phasen, an denen Palladium oder Platin beteiligt ist, sind bekannt. Sie haben eine kubisch-flächenzentrierte, kubisch-innenzentrierte oder auch tetragonale Struktur, wobei auch sogenannte Überstrukturen auftreten können. Solche Phasen werden z. B. in Gmelins Handbuch der Anorganischen Chemie, 8. Aufl., Bd. 68, Teil A, S. 617 – 652 (1951), beschrieben.

Die nach der Erfindung zu verwendenden intermetallischen Palladium-Kupfer- oder Platin-Kupfer-Katalysatoren werden z. B. erhalten, indem man die, wie oben angegeben, auf an sich bekannte Weise hergestellten Katalysatoren auf 400 bis 900° C, vorzugsweise 500 bis 800° C, erhitzt. Im allgemeinen steht dabei die Dauer des Erhitzens und die Höhe der erreichten Temperatur in einem gewissen Zusammenhang. Es hat sich als zweckmäßig erwiesen, z. B. 15 Minuten bis 4 Stunden auf 400 bis 900° C zu erhitzen, wobei im allgemeinen die kürzere Erhitzungszeit der höheren Temperatur zugeordnet ist. Da sich beim Erhitzen schließlich eine gewisse Stabilisierung des einmal erreichten katalytisch wirksamen Zustandes einstellt, ist die Anwendung einer bestimmten Höchstzeit im allgemeinen nicht erforderlich. Falls der Träger oder die Metalle unter den Bedingungen des Erhitzens zur Oxidation neigen, wird man zweckmäßig das Erhitzen in Gegenwart einer reduzierenden Atmosphäre, z. B. in reinem Wasserstoff oder eines Inertgases wie Stickstoff vornehmen. In dem so behandelten Katalysator läßt sich das Vorliegen der intermetallischen Palladium-Kupfer- oder Platin-Kupfer-Verbindung durch eine Röntgenstrukturanalyse leicht feststellen.

Als besonders wirksam erwiesen sich Katalysatoren mit kubisch-flächenzentrierter Kirstallstruktur, wie sie beispielsweise für die Verbindung $PdCu_3$ gefunden wird. Es hat sich jedoch gezeigt, daß es nicht erforderlich ist, die zu diesen Verbindungen führenden Ausgangsstoffe in stöchiometrischem Verhältnis einzusetzen. Wegen der Breite der entsprechenden Phasengebiete ist die Zusammensetzung in weiten Grenzen variierbar, wobei das Kristallgitter je nach Bedingungen Überstrukturen aufweisen oder auch verzerrt sein kann. Die Bestimmung der Gitterstruktur der intermetallischen Verbindungen kann in Anwesenheit der Trägerstoffe erfolgen. Auch die Kristall(it)größe der wirksamen Verbindungen kann einen gewissen Einfluß auf die katalytische Aktivität ausüben. So wird die Aktivität z. B. durch eine möglichst kleine Kristallitgröße günstig beeinflußt.

Als ggf. substituiertes Butadien kann man für das erfindungsgemäße Verfahren außer Butadien auch andere konjugierte Diolefine, wie Isopren, 2,3-Dimethyl-1,3-butadien, 1,3-Pentadiene, wie Piperylen oder durch Acyloxygruppen substituierte 1,3-Butadiene, wie 1-Acetoxybutadien, 1-Acetoxy-2-methyl-1.3-butadien oder 1-Acetoxy-3-methyl-1.3-butadien verwenden. Die genannten Diolefine können für sich oder als Mischungen, die z. B. auch noch andere Kohlenwasserstoffe, wie Monoolefine und Paraffinkohlenwasserstoffe enthalten, eingesetzt werden. Solche Mischungen stehen z. B. als sogenannte $C_4$-Schnitte zur Verfügung. Als niedermolekulare Carbonsäuren kommen z. B. Fettsäuren mit 1 bis 3 C-Atomen, wie Ameisensäure, Essigsäure oder Propionsäure in Betracht.

Die Umsetzung zur Herstellung der Acyloxybutene nimmt man in der Gas- oder Flüssigphase bei

3

Temperaturen von 70 bis 180°C vor. In der Gasphase arbeitet man vorzugsweise bei 120 bis 150°C und in der Flüssigphase vorzugsweise bei 70 bis 110°C. Der Reaktionsdruck ist durch die Verfahrensweise gegeben und kann zwischen atmosphärischem Druck und z. B. 100 bar betragen. Das Verfahren kann chargenweise oder kontinuierlich z. B. mit fixiertem Bett, Wirbelbett oder Dreiphasenfließbett durchgeführt werden.

Die nach dem Verfahren der Erfindung herstellbaren Acyloxybutene sind wertvolle Zwischenprodukte, z. B. für die Herstellung von Butendiol-1.4, Butandiol-1.4 und Tetrahydrofuran. Das in untergeordneten Mengen gebildete Butendiol-3.4-diacetat (Vinylglykoldiacetat) ist als Zwischenprodukt zur Herstellung von Vitaminen und anderen biologisch wirksamen Verbindungen geeignet.

Die bei der Acetoxilierung von Isopren oder 1-Acetoxy-2-methyl-1.3-butadien erhaltenen 2-Methyl-1.4-diacetoxy-2-butene oder 1.1.4-Triacetoxy-2-methyl-2-butene sind wertvolle Zwischenprodukte z. B. für die Synthese von Terpenverbindungen.

Bei dem erfindungsgemäßen Verfahren werden die gewünschten Acyloxybutene in hoher Ausbeute gebildet. Wegen der hohen Selektivität und der erhöhten Standzeit der verwendeten Katalysatoren werden höhere Raum-Zeit-Ausbeuten als bei den obengenannten bekannten Verfahren erhalten. Dieses Ergebnis ist überraschend, da man bei Verwendung von Palladium oder Platin und Kupfer sowie Tellur enthaltenden Katalysatoren, die keine intermetallische Phasen aufweisen, erheblich ungünstigere Ergebnisse erhält.

Im experimentellen Teil werden folgende Abkürzungen benutzt:

trans-1.4-Diacetoxy-2-buten   = trans-1.4-BEDA
cis-1.4-Diacetoxy-2-buten     = cis-1.4-BEDA
3.4-Diacetoxy-1-buten         = 3.4-BEDA
3.4-Hydroxyacetoxy-1-butene   = 3.4-BEMA
1.4-Hydroxyacetoxy-2-butene   = 1.4-BEMA

Bei den Prozentangaben handelt es sich um Gewichtsprozente.

## Beispiel 1

### a) Katalysatorherstellung

13,5 g Kupferpulver, gelöst in 100 cm$^3$ 33%iger Salpetersäure, werden bei Raumtemperatur mit 12,51 g PdCl$_2$, gelöst in 60 cm$^3$ eines warmen Gemisches aus 66%iger Salpetersäure und 32%iger Salzsäure (Volumenverhältnis 1 : 1) und 0,19 g TeO$_2$ in 30 cm$^3$ warmer, 16%iger Salzsäure, vermischt. Man legt 100 g Aktivkohle (0,3 bis 0,5 mm) vor, die zuvor bei 70°C 5 Stunden lang mit 15%iger Salpetersäure gerührt, nach dem Abnutschen neutral gewaschen und bei 150°C unter Vakuum getrocknet worden war (Schwefelgehalt 0,21%) und fügt die vereinigte Metallsalzlösung hinzu. Anschließend gibt man soviel Wasser hinzu, daß die Kohle völlig benetzt ist. Dann wird am Rotationsverdampfer bei 85°C und Wasserstrahlvakuum zur Trockene eingedampft. Der Katalysator wird 2 Stunden bei 150°C im Vakuumtrockenschrank, dann 2 Stunden bei 150°C im Röhrenofen unter strömendem Stickstoff getrocknet. Anschließend wird 6 Stunden bei 200°C, dann 6 Stunden bei 400°C mit bei Raumtemperatur mit Methanol gesättigtem Stickstoff und schließlich 0,5 Stunden mit Wasserstoff (20 l/Stunde) bei 800°C aktiviert. Man läßt unter strömendem Stickstoff auf Raumtemperatur abkühlen.

Nach der Elementaranalyse enthält der Katalysator 5,36% Palladium, 9,39% Kupfer und 0,11% Tellur. Das Grammatomverhältnis von Pd : Cu : Te beträgt 1 : 2,9 : 0,02. Die Röntgenanalyse zeigt eine PdCu$_3$-Phase, die praktisch kein PdCu enthält. Beugungslinien, die Pd-Te-Phasen entsprechen könnten, werden nicht beobachtet. Die Gitterkonstante der PdCu$_3$-Phase beträgt 3,6934 Å, die mittlere Kristallitgröße 234 Å. Die gemessenen d-Werte in nm (CuK$_{\bar{a}}$-Strahler) sind in der nachfolgenden Tabelle angegeben.

| PdCu$_3$ | (hkl) |
|---|---|
| 0,37 | 001 |
| 0,26 | 011 |
| 0,213 | 111 |
| 0,1848 | 002 |
| 0,1305 | 022 |

### b) Acetoxylierung von Butadien

In einem 1-Liter-Dreihalskolben mit Anschützaufsatz, Tropftrichter, Begasungsrührer, Innenthermometer, Gaseinleitungsrohr und Rückflußkühler mit aufgesetztem Trockeneiskühler werden nach dem Spülen der Apparatur mit Stickstoff 25 g des nach Beispiel 1a) hergestellten Katalysators (5,36% Pd, 9,39% Cu, 0,11% Te), suspendiert in 543 g Eisessig auf 95°C erhitzt. Im Verlauf von 4 Stunden werden gleichzeitig je 12 l Sauerstoff und Butadien eingeleitet. Nach beendeter Zugabe wird bei 95°C 30 Minuten lang Stickstoff durch das Reaktionsgemisch geleitet. Man läßt abkühlen und entfernt den Katalysator durch Filtrieren. Die anfallende Essigsäurelösung (579 g) enthält nach der GC-Analyse 9,37% trans-1.4-BEDA, 1,07% cis-1.4-BEDA, 1,42% 3.4-BEDA, 0,09% 3.4-BEMA und 0,01% 1.4-BEMA. Demnach sind insgesamt 69,2 g Butendioldiacetate und -monoacetate entstanden.

### Vergleichsbeispiel 1

### a) Katalysatorherstellung

Aus 150 g der in Beispiel 1a) genannten Aktivkohle, 12,51 g PdCl$_2$ und 0,19 g TeO$_2$ wird nach den Angaben von Beispiel 26 der DE-OS 2 217 452 ein Pd-Te-Katalysator hergestellt. Der Katalysator wird wie beschrieben 2 Stunden bei 200°C und 1 Stunde bei 400°C mit Methanol aktiviert. Die Elementaranalyse ergibt einen Gehalt von 4,90% Palladium und 0,11% Tellur. Die Röntgenanalyse zeigt keine Beugungslinien, die einer intermetallischen Palladium-Tellur-Phase, wie z. B. Pd$_4$Te entsprochen hätte.

### b) Acetoxylierung von Butadien

In gleicher Weise wie in Beispiel 1b) beschrieben, wird Butadien in Gegenwart von 25 g des nach Vergleichsbeispiel 1a) hergestellten Katalysators (4,90% Pd, 0,11% Te) mit Eisessig und Sauerstoff umgesetzt. Es werden 555 g Essigsäurelösung folgender Zusammensetzung erhalten (GC-Analyse): 0,65% trans-1.4-BEDA, 0,19% cis-1.4-BEDA und 0,1% 3.4-BEDA. Insgesamt werden nur 5,2 g Butendioldiacetate und -monoacetate gebildet.

### Beispiel 2

Wie in Beispiel 1a) beschrieben, werden 400 g eines Pd-Cu-Te-Katalysators (enthaltend: 4,67% Pd, 6,7% Cu, 0,67% Te bei einem Grammatomverhältnis von Pd : Cu : Te von 1 : 2,4 : 0,12) zunächst ohne die halbstündige Aktivierung bei 800°C mit Wasserstoff hergestellt. Anschließend werden Einzelchargen von 60 g dieses Katalysators jeweils 0,5 Stunden lang bei 600 bis 800°C wie in Beispiel 1a) beschrieben mit Wasserstoff aktiviert. Die Tabelle 1 zeigt die Ergebnisse der Acetoxylierung mit je 25 g dieser Katalysatoren, die analog Beispiel 1b) durchgeführt wird.

Tabelle 1

| Bei-spiel | Temperatur der H$_2$-Aktivierung | Reaktions-austrag | trans-1.4-BEDA | cis-1.4-BEDA | 3.4-BEDA | 1.4-BEMA | 3.4-BEMA | BEDA + BEMA |
|-----------|----------------------------------|--------------------|----------------|--------------|----------|----------|----------|-------------|
| | [°C] | [g] | [Gew.-%] | | | | | [g] |
| 2a | keine H$_2$-Aktivierung | 547 | 1,51 | 0,16 | 0,37 | 0,007 | 0,14 | 12,0 |
| 2b | 600 | 564 | 5,59 | 0,91 | 0,91 | 0,003 | 0,01 | 41,8 |
| 2c | 700 | 567 | 6,40 | 1,02 | 0,99 | — | 0,02 | 47,8 |
| 2d | 800 | 574 | 8,62 | 1,39 | 1,28 | 0,004 | 0,04 | 65,0 |

Die Aktivierung des zunächst bei 400°C mit Methanol behandelten Katalysators wird bei 800°C 0,5 Stunden lang mit Stickstoff (20 l/Stunde) anstelle von Wasserstoff durchgeführt. Nach der Acetoxylierung analog Beispiel 1b) in Gegenwart von 25 g Katalysator sind in 571 g Reaktionsaustrag erhalten: 8,06% trans-1.4-BEDA, 1.31% cis-1.4-BEDA, 1,20% 3.4-BEDA, 0,005% 1.4-BEMA, 0,03% 3.4-BEMA. Insgesamt sind also 60,5 g Butendioldiacetate und -monoacetate entstanden.

## Vergleichsbeispiel 2

25 g eines nach Beispiel 26 der DE-OS 2 217 452 hergestellten Palladium-Tellur-Katalysators, der 4,9% Pd und 0,89% Te enthält, werden — zusätzlich zu der dort beschriebenen Aktivierung mit Methanol — 0,5 Stunden lang bei 800°C mit Wasserstoff (20 l/Stunde) aktiviert.

Bei der analog Beispiel 1b) durchgeführten Acetoxylierung von Butadien werden 561 g einer Essigsäurelösung erhalten, die nach der GC-Analyse 4,47% trans-1.4-BEDA, 0,87% cis-1.4-BEDA und 0,78% 3.4-BEDA enthält. Insgesamt werden also nur 34,3 Teile Butendioldiacetate und -monoacetate gefunden.

## Beispiel 3

In einem 1-Liter-Dreihalskolben, der mit Rührer, Rückflußkühler und Innenthermometer mit Anschützaufsatz versehen ist, werden 25 g eines Katalysators, der nach Beispiel 1a) hergestellt wird und 5,31% Pd, 8,9% Cu und 0,51% Te enthält (Grammatomverhältnis Pd : Cu : Te wie 1 : 2,8 : 0,08) in 600 g Eisessig suspendiert. Unter intensivem Rühren werden bei 95°C pro Stunde je 3 l Butadien und Sauerstoff und 200 g Eisessig zugeleitet. Über ein Steigrohr mit Fritte wird das Reaktionsprodukt in Sammelgefäßen aufgefangen, deren Inhalt regelmäßig analysiert wird. Nach einer Reaktionszeit von 200 Stunden werden so 1.232 g Butendioldiacetate und -monoacetate gewonnen. Das durchschnittliche Molverhältnis der gebildeten Diacetate beträgt trans-1.4-BEDA : cis-1.4-BEDA : 3,4-BEDA = 1 : 0,15 : 0,14.

## Vergleichsbeispiel 3

Anstelle des in Beispiel 3 genannten Katalysators verwendet man einen Kontakt, der 5,1% Pd und 0,45% Te enthält und wie in Beispiel 26 der DE-OS 2 217 452 beschrieben, hergestellt wird. Die Reaktion wird mit gleichen Stoffmengen und unter völlig gleichen Bedingungen wie in Beispiel 3 beschrieben durchgeführt. Innerhalb von 200 Betriebsstunden werden nur 423,5 g Butendioldiacetate und -monoacetate erhalten. Das durchschnittliche Molverhältnis der gebildeten Diacetate beträgt trans-1.4-BEDA : cis-1.4-BEDA : 3.4-BEDA = 1 : 0,49 : 0,15.

## Beispiel 4

Ein 4 m langes Edelstahlrohr mit einem Innendurchmesser von 45 mm wird mit 800 g eines nach Beispiel 1a hergestellten Katalysators gefüllt, der 5,2% Pd, 8,3% Cu und 0,5% Te (Grammatomverhältnis Pd : Cu : Te wie 1 : 2,7 : 0,08) enthält und mit Glas-Raschigringen vermischt ist.

Durch das Rohr werden bei 95°C und einem Druck von 70 bar pro Stunde 1,2 l flüssiger $C_4$-Crackschnitt, der 25% Butadien enthält und 25 Nl Sauerstoff, gelöst in 5 l Eisessig, geleitet. Die durchschnittliche Konzentration an Butendioldiacetaten und -monoacetaten im Reaktionsaustrag beträgt ohne abzusinken über einen Zeitraum von 400 Stunden 6,05 Gew.-% (88,5% 1.4- und 11,5% 3.4-Isomere). Die Selektivität beträgt 94,2%. Insgesamt werden innerhalb der 400 Stunden 121 kg Butendioldiacetate und -monoacetate gebildet. Nach der Elementaranalyse des gebrauchten Katalysators sind nach 400 Stunden rund 47% des ursprünglich auf der Aktivkohle befindlichen Kupfers in Lösung gegangen.

## Vergleichsbeispiel 4a

In Gegenwart von 440 g eines Pd-Cu-Katalysators (5,3% Pd, 7,1% Cu), der unter Weglassen des $TeO_2$ nach Beispiel 1a) hergestellt wird, setzt man in der gleichen Apparatur unter gleichen Bedingungen und mit gleichen Stoffmengen wie im Beispiel 4 beschrieben $C_4$-Crackschnitt mit Sauerstoff und Essigsäure um. Die Katalysatoraktivität sinkt innerhalb von 70 Stunden praktisch auf Null. Die in diesem Zeitraum produzierte Menge an Butendioldiacetaten und -monoacetaten beträgt nur 4,6 kg. 94% des ursprünglich auf dem Katalysator vorhandenen Kupfers sind in Lösung gegangen.

6

### Vergleichsbeispiel 4b

Entsprechend der in Beispiel 1 der DE-OS 2 728 574 beschriebenen Katalysatorherstellung werden 100 g der in Beispiel 1a genannten, mit Salpetersäure vorbehandelten Aktivkohle (0,21% Schwefel) zu einer Lösung von 13,4 g Dischwefeldichlorid in 400 cm³ Schwefelkohlenstoff gegeben. Das Reaktionsgemisch wird am Rotationsverdampfer zur Trockene eingedampft.

8,96 g Kupferpulver, gelöst in 66 cm³ 33%iger Salpetersäure werden bei Raumtemperatur mit 8,34 g PdCl, gelöst in 40 cm³ eines warmen Gemisches aus 66%iger Salpetersäure und 32%iger Salzsäure (Volumenverhältnis 1 : 1) und 0,63 g TeO₂ in 100 cm³ warmer 16%iger Salzsäure vermischt. Die Salzlösung wird zur Aktivkohle hinzugegeben und es wird bei der weiteren Katalysatorherstellung und -aktivierung wie in Beispiel 1a beschrieben verfahren.

Mit 25 g des so hergestellten Katalysators wird die Acetoxylierung von Butadien wie in Beispiel 1b beschrieben durchgeführt. In den dabei erhaltenen 560 g Reaktionslösung sind nur 4,58% trans-1.4-BEDA, 0,66% cis-BEDA, 0,75% 3.4-BEDA, 0,004% 1.4-BEMA und 0,02% 3.4-BEMA, insgesamt also 33,6 g Butendioldiacetate und -monoacetate enthalten.

### Vergleichsbeispiel 4c

Entsprechend Vergleichsbeispiel 4b wird ein Pd-Cu-Katalysator hergestellt, wobei man anstelle von 100 g Aktivkohle die gleiche Menge Silika-Gel (Grace, Typ 113, 1 – 3 mm Körnung) verwendet und das TeO₂ fortläßt. Die nach Beispiel 1b in Gegenwart von 25 g Katalysator durchgeführte Acetoxylierung ergibt in 548 g Reaktionsaustrag nur 0,076% trans-1.4-BEDA, 0,009% cis-1.4-BEDA und 0,007% 3.4-BEDA.

Wird die Aktivierung des Katalysators nur bei 400°C mit Methanol durchgeführt, so sind nach der Acetoxylierung in 546 g Reaktionsaustrag nur 0,11% trans-1.4-BEDA, 0,01% cis-1.4-BEDA und 0,02% 3.4-BEDA enthalten.

### Beispiele 5 bis 11

In gleicher Weise wie in Beispiel 1a beschrieben, werden die in Tabelle 2 zusammengestellten Pd-Cu-Te-Katalysatoren hergestellt. Die Acetoxylierungsversuche werden mit gleichen Stoffmengen und unter gleichen Bedingungen, wie in Beispiel 1b beschrieben, durchgeführt. Man erhält die in der Tabelle 2 angegebenen Ergebnisse.

Tabelle 2

| Beispiel Nummer | Katalysatorzusammensetzung | | | Reaktions-austrag | trans-1.4-BEDA | cis-1.4-BEDA | 3.4-BEDA | 1.4-BEMA | 3.4-BEMA | BEDA + BEMA |
| --- | --- | --- | --- | --- | --- | --- | --- | --- | --- | --- |
| | Pd | Cu | Te | | | | | | | |
| | [Gew.-%] (Grammatomverhältnis) | | | [g] | [Gew.-%] | | | | | [g] |
| 5*) | 5,17 (1) | 8,30 (2,7) | 0,55 (0,09) | 574 | 8,83 | 1,19 | 1,37 | 0,009 | 0,16 | 66,3 |
| 6 | 4,84 (1) | 12,9 (4,5) | 1,1 (0,19) | 565 | 6,04 | 0,97 | 0,89 | 0,03 | 0,02 | 44,9 |
| 7 | 5,54 (1) | 6,76 (2) | 0,1 (0,02) | 575 | 8,47 | 1,15 | 1,39 | 0,006 | 0,04 | 63,6 |
| 8 | 5,29 (1) | 2,95 (0,9) | 1,1 (0,17) | 563 | 5,57 | 0,94 | 1,13 | 0,004 | 0,08 | 43,5 |
| 9 | 2,55 (1) | 8,7 (5,8) | 0,71 (0,23) | 559 | 6,60 | 0,76 | 1,10 | 0,006 | 0,22 | 48,6 |
| 10 | 2,75 (1) | 5,43 (3,3) | 0,25 (0,08) | 581 | 10,93 | 1,61 | 1,60 | 0,009 | 0,19 | 83,3 |
| 11 | 4,97 (1) | 8,3 (2,8) | 1,7 (0,28) | 569 | 7,44 | 1,13 | 1,06 | 0,002 | — | 54,8 |

*) 1 Stunde mit Wasserstoff bei 800°C aktiviert.

**0 027 979**

Beispiel 12

In Gegenwart von 25 g des nach Beispiel 1a hergestellten Pd-Cu-Te-Katalysators (5,36% Pd, 9,39% Cu, 0,11% Te) und 543 g Eisessig werden wie in Beispiel 1b beschrieben innerhalb von 4 Stunden 34 g Isopren mit 12 l Sauerstoff umgesetzt. Im Reaktionsaustrag (583 g) sind nach der GC-Analyse 8,35% 2-Methyl-1.4-diacetoxy-2-buten (26% cis, 74% trans) enthalten.

Beispiel 13

Wird Beispiel 12 mit der gleichen Menge Piperylen anstelle von Isopren wiederholt, so sind nach der GC-Analyse im Reaktionsaustrag (580 g) 3,95% 1-Methyl-1.4-diacetoxy-2-butene enthalten.

Beispiel 14

Wird Beispiel 12 mit 63 g 1-Acetoxy-2-methyl-1.3-butadien anstelle von Isopren in Gegenwart von 25 g des in Beispiel 3 genannten Katalysators wiederholt, so sind nach der GC-Analyse im Reaktionsaustrag (610 g) 5,84% cis + trans-1.1.4-Triacetoxy-2-methyl-2-buten, 1,23% cis + trans-4-Acetoxytiglinaldehyd, 3,36% unumgesetztes 1-Acetoxy-2-methyl-1.3-butadien und 0,37% Tiglinaldehyd enthalten.

Beispiel 15

a) Katalysatorherstellung

3,66 g Kupferpulver, gelöst in 25 cm$^3$ 33%iger Salpetersäure, werden bei Raumtemperatur mit 6,48 g PtCl$_4$, gelöst in 20 cm$^3$ eines warmen Gemisches aus 66%iger Salpetersäure und 32%iger Salzsäure (Volumenverhältnis 1 : 1) und 0,95 g TeO$_2$ in 50 cm$^3$ warmer, 16%iger Salzsäure, vermischt. Man legt 150 g Aktivkohle (0,3 bis 0,5 mm) vor, die zuvor bei 70°C 5 Stunden lang mit 15%iger Salpetersäure gerührt, nach dem Abnutschen neutral gewaschen und bei 150°C unter Vakuum getrocknet worden war (Schwefelgehalt 0,27%) und fügt die vereinigte Metallsalzlösung hinzu. Anschließend gibt man soviel Wasser hinzu, daß die Kohle völlig benetzt ist. Dann wird am Rotationsverdampfer bei 85°C und Wasserstrahlvakuum zur Trockene eingedampft. Der Katalysator wird 4 Stunden bei 150°C im Röhrenofen unter strömendem Stickstoff getrocknet. Anschließend wird 6 Stunden bei 200°C, dann 6 Stunden bei 400°C mit bei Raumtemperatur mit Methanol gesättigtem Stickstoff und schließlich 0,5 Stunden mit Wasserstoff (20 l/Stunde) bei 800°C aktiviert. Man läßt unter strömendem Stickstoff auf Raumtemperatur abkühlen.

Nach der Elementaranalyse enthält der Katalysator 2,5% Platin, 2,4% Kupfer, und 0,5% Tellur. Das Grammatomverhältnis von Pt : Cu : Cu beträgt 1 : 2.95 : 0,3. Die Röntgenanalyse zeigt eine kubisch-flächenzentrierte Phase, die strukturell PdCu$_3$ gleicht. Beugungslinien, die Pt-Te-Phasen entsprechen könnten, werden nicht beobachtet. Die Gitterkonstante der Pt-Cu-Phase beträgt 0,369 nm, die mittlere Kristallitgröße etwa 10 nm; die gemessenen d-Werte in nm (CuK$^-_\alpha$-Strahler) bis d = 0,12 nm sind in der nachfolgenden Tabelle angegeben.

| d (nm) | (hkl) |
|--------|-------|
| 0,369 | 001 |
| 0,261 | 011 |
| 0,213 | 111 |
| 0,184 | 002 |
| 0,165 | 012 |
| 0,151 | 112 |
| 0,1307 | 022 |

9

### b) Acetoxylierung von Butadien

In gleicher Weise wie in Beispiel 1b beschrieben, wird Butadien in Gegenwart von 25 g des nach Beispiel 15a hergestellten Katalysators (2,5% Pt, 2,4% Cu, 0,5% Te) mit Eisessig und Sauerstoff umgesetzt. Es werden 556 g Essigsäurelösung folgender Zusammensetzung erhalten (GC-Analyse): 3,0% trans-1,4-BEDA, 0,94% cis-1,4-BEDA und 1,07% 3,4-BEDA. Insgesamt werden 27,8 g Butendiolacetate gefunden.

### Vergleichsbeispiel 15

### a) Katalysatorherstellung

Wie in Beispiel 15a beschrieben, aber unter Weglassen des Kupferpulvers und der zum Lösen benötigten Salpetersäure, wird ein Pt-Te-Katalysator hergestellt, der nach der Elementaranalyse 2,5% Platin und 0,5% Tellur enthält. Röntgenographisch lassen sich nur die Beugungslinien von metallischem Platin nachweisen.

### b) Acetoxylierung von Butadien

In gleicher Weise wie in Beispiel 1b beschrieben, wird Butadien in Gegenwart von 25 g des nach Vergleichsbeispiel 15a hergestellten Katalysators (2,5% Pt, 0,5% Te) mit Eisessig und Sauerstoff umgesetzt. Es werden 547 g Essigsäurelösung folgender Zusammensetzung erhalten (GC-Analyse): 0,45% trans-1,4-BEDA, 0,12% cis-1,4-BEDA und 0,13% 3,4-BEDA. Insgesamt werden 3,8 g Butendioldiacetate gefunden.

### Beispiel 16

Wiederholt man Beispiel 1b mit 543 g Propionsäure anstelle von Eisessig, so erhält man nach der Acetoxylierung 570 g Reaktionslösung. Nach Abziehen der Propionsäure am Rotationsverdampfer und fraktionierter Destillation des Rückstandes gewinnt man daraus 56,8 g Butendioldipropionate und -monopropionate vom Siedepunkt 82 bis 88° C/0,5 mbar, $n_D^{20} = 1,4420$.

**Patentansprüche**

1. Verfahren zur Herstellung von Acyloxybutenen durch Umsetzung von ggf. substituiertem 1.3-Butadien mit Sauerstoff und niedermolekularen Carbonsäuren in Gegenwart eines Palladium oder Platin und Tellur enthaltenden Trägerkatalysators, dadurch gekennzeichnet, daß der Katalysator eine intermetallische Palladium-Kupfer- oder Platin-Kupfer-Verbindung enthält.

2. Verfahren nach Anspruch 1, dadurch gekennzeichnet, daß der Katalysator eine intermetallische Palladium-Kupfer-Verbindung enthält.

3. Verfahren nach Anspruch 1, dadurch gekennzeichnet, daß der Katalysator eine intermetallische Platin-Kupfer-Verbindung enthält.

4. Verfahren nach Anspruch 1, dadurch gekennzeichnet, daß man einen Trägerkatalysator verwendet, der, bezogen auf das Katalysatorgewicht 1 bis 10% Palladium oder Platin, 0,1 bis 30% Kupfer und 0,01 bis 10% Tellur enthält.

5. Verfahren nach Anspruch 1, dadurch gekennzeichnet, daß man einen Trägerkatalysator verwendet, der je Grammatom Palladium oder Platin 0,01 bis 6 Grammatome Kupfer und 0,01 bis 1 Grammatome Tellur enthält.

6. Verfahren nach Anspruch 1, dadurch gekennzeichnet, daß man einen Trägerkatalysator verwendet, der je Grammatom Palladium oder Platin 1 bis 3,5 Grammatome Kupfer und 0,01 bis 0,4 Grammatome Tellur enthält.

7. Verfahren nach Anspruch 1, dadurch gekennzeichnet, daß man als gegebenenfalls substituiertes 1,3-Butadien das nicht substituierte 1,3-Butadien, Isopren oder 1-Acetoxy-2-methyl-1,3-butadien verwendet.

**Claims**

1. A process for the preparation of acyloxybutenes by reacting optionally substituted 1,3-butadiene with oxygen and a low molecular weight carboxylic acid in the presence of a supported catalyst comprising palladium or platinum and tellurium, characterized in that the catalyst contains an intermetallic palladium-copper or platinum-copper compound.

2. A process as claimed in claim 1, characterized in that the catalyst contains an intermetallic palladium-copper compound.

3. A process as claimed in claim 1, characterized in that the catalyst contains an intermetallic platinum-copper compound.

4. A process as claimed in claim 1, characterized in that a supported catalyst is used which contains, based on the catalyst weight, from 1 to 10% of palladium or platinum, from 0.1 to 30% of copper and from 0.01 to 10% of tellurium.

5. A process as claimed in claim 1, characterized in that a supported catalyst is used which contains, per gram-atom of palladium or platinum, from 0.01 to 6 gram-atoms of copper and from 0.01 to 1 gram-atom of tellurium.

6. A process as claimed in claim 1, characterized in that a supported catalyst is used which contains, per gram-atom of palladium or platinum, from 1 to 3.5 gram-atoms of copper and from 0.01 to 0.4 gram-atom of tellurium.

7. A process as claimed in claim 1, characterized in that the optionally substituted 1,3-butadiene used is unsubstituted 1,3-butadiene, isoprene or 1-acetoxy-2-methyl-1,3-butadiene.


**Revendications**

1. Procédé de préparation d'acyloxybutènes par réaction de butadiène-1,3 éventuellement substitué avec l'oxygène et des acides carboxyliques à bas poids moléculaire en présence d'un catalyseur au palladium ou au platine et au tellure sur support, caractérisé en ce que le catalyseur contient un composé intermétallique palladium-cuivre ou platine-cuivre.

2. Procédé suivant la revendication 1, caractérisé en ce que le catalyseur contient un composé intermétallique palladium-cuivre.

3. Procédé suivant la revendication 1, caractérisé en ce que le catalyseur contient un composé intermétallique platine-cuivre.

4. Procédé suivant la revendication 1, caractérisé en ce que l'on emploie un catalyseur sur support qui contient 1 à 10% de palladium ou de platine, 0,1 à 30% de cuivre et 0,01 à 10% de tellure par rapport au poids du catalyseur.

5. Procédé suivant la revendication 1, caractérisé en ce que l'on emploie un catalyseur sur support qui contient 0,01 à 6 atomes-grammes de cuivre et 0,01 à 1 atome-gramme de tellure par atome-gramme de palladium ou de platine.

6. Procédé suivant la revendication 1, caractérisé en ce que l'on emploie un catalyseur sur support qui contient 1 à 3,5 atomes-grammes de cuivre et 0,10 à 0,4 atome-gramme de tellure par atome-gramme de palladium ou de platine.

7. Procédé suivant la revendication 1, caractérisé en ce que l'on emploie comme butadiène-1,3-éventuellement substitué du butadiène-1,3 non substitué, l'isoprène ou l'acétoxy-1 méthyl-2 butadiène-1,3.